(19) Europäisches Patentamt
European Patent Office
Office européen des brevets

(11) **EP 3 332 341 B1**

(12) **EUROPEAN PATENT SPECIFICATION**

(45) Date of publication and mention
of the grant of the patent:
**19.11.2025 Bulletin 2025/47**

(51) International Patent Classification (IPC):
**G16C 20/30** (2019.01)    **G16C 20/50** (2019.01)
**G16C 20/80** (2019.01)

(21) Application number: **16745734.0**

(52) Cooperative Patent Classification (CPC):
**G16C 20/50; G16C 20/30; G16C 20/80**

(22) Date of filing: **29.07.2016**

(86) International application number:
**PCT/EP2016/068248**

(87) International publication number:
**WO 2017/025361 (16.02.2017 Gazette 2017/07)**

(54) **METHOD, COMPUTER PROGRAM AND SYSTEM FOR OPTIMIZING A MOLECULE FOR MEDICAL APPLICATIONS**

VERFAHREN, COMPUTERPROGRAMM UND SYSTEM ZUR OPTIMIERUNG EINES MOLEKÜLS FÜR MEDIZINISCHE ANWENDUNGEN

PROCÉDÉ, PROGRAMME INFORMATIQUE ET SYSTÈME POUR OPTIMISER UNE MOLÉCULE POUR APPLICATIONS MÉDICALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.08.2015 EP 15180251**
**16.12.2015 EP 15200519**

(43) Date of publication of application:
**13.06.2018 Bulletin 2018/24**

(73) Proprietor: **VeriSIM Life, Inc.**
**San Francisco CA 94104 (US)**

(72) Inventors:
• **CINCILLA, Giovanni**
**08035 Barcelona (ES)**
• **MASONI, Simone**
**08015 Barcelona (ES)**
• **BLOBEL, Jascha Urs**
**08015 Barcelona (ES)**

(74) Representative: **Schulz Junghans**
**Patentanwälte PartGmbB**
**Großbeerenstraße 71**
**10963 Berlin (DE)**

(56) References cited:
**WO-A1-2014/147744       WO-A1-2015/061602**
**US-A1- 2011 237 458**

• SMITHERS T. ET AL: "An approach to intelligent drug design support", SYSTEM SCIENCES, 1993, PROCEEDING OF THE TWENTY-SIXTH HAWAII INTERNATI ONAL CONFERENCE ON WAILEA, HI, USA 5-8 JAN. 1993, LOS ALAMITOS, CA, USA,IEEE, US, vol. i, 5 January 1993 (1993-01-05), pages 634 - 645, XP010640427, ISBN: 978-0-8186-3230-3, DOI: 10.1109/ HICSS.1993.270677
• SCHNEIDER G. ET AL: "Computer-based de novo design of drug-like molecules", NATURE REVIEWS. DRUG DISCOVERY, NATURE PUBLISHING GROUP, GB, vol. 4, no. 8, 1 August 2005 (2005-08-01), pages 649 - 663, XP002435753, ISSN: 1474-1784, DOI: 10.1038/ NRD1799
• WOLBER G. ET AL: "Molecule-pharmacophore superpositioning and pattern matching in computational drug design", DRUG DISCOVERY TODAY, ELSEVIER, RAHWAY, NJ, US, vol. 13, no. 1-2, 5 November 2007 (2007-11-05), pages 23 - 29, XP022413777, ISSN: 1359-6446, DOI: 10.1016/ J.DRUDIS.2007.09.007

EP 3 332 341 B1

**Description**

The invention relates to a method, a computer program, a system, a video game system and a video game for designing a molecule for medical applications by optimization of an associated drug score of the molecule.

In drug discovery and development a major challenge is to identify new molecules that are on the one hand capable to act through a specific mechanism of action, e.g. bind to a or more certain biological targets in order to activate or inhibit said biological target, and that are on the other hand compatible with the physiological constrains set by e.g. the human body. These constraints comprise for example side effects, allergic or even toxic reactions etc.

One approach to design such medical molecules is to provide so-called pharmacophores and to optimize based on these pharmacophores preferably *in silico* molecular structures such that binding/activation or inhibition is achieved with ideally almost no side effects and high selectivity.

A pharmacophore represents common molecular features of ligands that are, for example, capable to bind to a biological target, such as a protein. A pharmacophore therefore represents essential molecular features for other molecules that are also thought to exhibit the capability of binding to the biological target.

A pharmacophore normally represents common molecular features for a single pharmacological property, as for example the binding to the biological target, and not for multiple pharmacological properties.

There are a variety of possibilities to represent a pharmacophore. It is possible to create one-dimensional, two-dimensional or three-dimensional pharmacophores, wherein with each dimension added, the computational complexity to find a matching ligand increases.

As the possibilities to modify molecular structures, by e.g. randomly replacing or rearranging atoms of a selected molecule, are nearly countless (in the order of $10^{60}$ for molecules with a molecular weight of up to 500 g/mol, see [1]), a random optimization approach does not compute in a relevant time scale, and storage space even for one-dimensional pharmacophores.

In order to nonetheless come up with suitable solutions in time, optimization algorithms are applied to this problem. However, these algorithms typically exploit only a local environment of the chemical space, namely around predefined starting structures. The chemical space is given by molecules comprising different atoms or differently arranged atoms that either exhibit better pharmacological properties or - which is more likely - worse pharmacological properties.

Pharmacological properties comprise many kinds of biological properties of molecules such as for example cosmetic, veterinarian, neutraceutical and/or agrochemical properties. Cosmetic properties are particularly properties associated to the healing or preserving of human beauty or appearance, and which might even share properties used for medical applications. Veterinarian properties on the other hand, refer to pharmacological properties related to animals, wherein nutraceutical properties relate to the pharmacological properties of food, drinks and dietary product. Agrochemical properties comprise properties related to herbicides, insecticides, fungicides, and other pesticides, plant growth regulators, fertilizers, and animal feed supplements.

Another way of optimizing a molecule for medical applications is to make educated guesses that are preferably carried out by a person skilled in the art that particularly has a professional background in optimization processes for molecules. However, this approach is comparably complicated and time consuming. SCHNEIDER G. et al. ("Computer-based de novo design of drug-like molecules", NATURE REVIEWS. DRUG DISCOVERY, NATURE PUBLISHING GROUP, GB, vol. 4, no. 8, 1 August 2005, pages 649-663) discloses computer-based de novo design methods for generating and optimizing drug-like molecules using multi-objective scoring functions, including QSAR models, to guide automated molecular generation. SMITHERS T. et al. ("An approach to intelligent drug design support", SYSTEM SCIENCES, 1993, PROCEEDING OF THE TWENTY-SIXTH HAWAII INTERNATIONAL CONFERENCE ON WAILEA, HI, USA 5-8 JAN. 1993, LOS ALAMITOS, CA, USA,IEEE, US, vol. i, pages 634-645) describes a conceptual framework for intelligent drug design support systems that assist human designers through knowledge-based guidance and visualization tools.

Therefore, the problem underlying the present invention is to provide a method, a computer program and a system allowing the optimization of a molecule that creates new medical compounds in a comparably short time.

This problem is solved by a method having the features of claim 1, a computer program having the features of claim 14 and a system having the features of claim 15. Preferred embodiments are stated in the sub claims.

According to claim 1, a method is disclosed for designing a molecule for medical applications by optimization of an associated drug score (101) of the molecule, wherein the method is a computer-implemented method performed by a computerized device comprising a processor, comprising the steps of:

    a) providing a computer-readable representation of a selected molecule (100) and a drug score (101) associated to the selected molecule (100),
    b) determining (201) a first moiety (110) of the selected molecule (100) and a ssecond moiety (120) of the selected molecule (100), wherein the selected molecule (100) consists of the first moiety (110) and second moiety (120),
    c) determining (202) a first moiety pharmacophore (130), wherein the first moiety (110) of the selected molecule (100) fits to the first moiety pharmacophore (130), and wherein the first moiety pharmacophore is determined from a single molecule only,

d) providing a graphical user interface (150),

e) displaying (204) a graphical representation of a selected part (131) of the first moiety pharmacophore (130) on the graphical user interface (150),

f) determining a starting point (160) on the graphical user interface (150) in relation to the graphical representation of the selected part (131) of the first moiety pharmacophore (130),

g) from the starting point (160), arranging graphical representations of molecular building blocks (170) on the graphical user interface (150), wherein the graphical representations of the molecular building blocks (170) are interconnected and form a graphical representation of a modified first moiety of a molecule (180),

h) assigning (207) the graphical representation of the modified first moiety of the molecule (180) to a modified first moiety (111) of the selected molecule (100),

i) determining (208) a modified molecule (190) consisting of the modified first moiety (111) and the ssecond moiety (120) of the selected molecule (100),

j) estimating (209) the associated drug score (101) for the modified molecule (190),

k) disclosing (210) the associated drug score (101) of the modified molecule (190).

The method according the invention is for example a computer-implemented method performed by a computerized device comprising a processor, for designing a molecule for medical applications by optimization of an associated drug score of the molecule.

A medical use or a medical application is to be understood in a broad sense, comprising for example also uses in nutraceuticals, cosmetic, veterinary and or agrochemistry. Also the term drug design is not limited to medical drugs, but can be understood also as design of nutraceuticals, design of cosmetics, design of veterinary medicine and/or design of molecules used in agrochemistry.

By providing a computer-readable representation of the selected molecule, it is possible to process, simulate and predict molecular properties and features of the selected molecule.

The size of the molecules considered for the method according to the invention particularly ranges between 5 and 300 atoms, preferably between 10 and 100 atoms. Such molecules are also referred to as small molecules. These molecules are particularly interesting for medical applications. Medical applications in this context refer preferably to medical applications for treating humans but also for veterinary and agrochemical purposes it is possible to apply the present invention.

A key feature of the method is to determine a drug score, e.g. a single number, such that it is readily recognizable whether the drug score is increasing or decreasing as compared to an initial drug score from the unmodified molecule. For example, the drug score may be a number within a specific range, e.g. in the range of [0-100] or [0-1]. However the representation of the drug score can be realized in various ways such as for example a visual representation by a color code or color scale, or an audible representation using sound indications.

It is advantageous to virtually divide the selected molecule into a first and a second moiety for a variety of reasons. One reason is that the first moiety can be chosen such that only a certain number of atoms or molecular features are comprised by said first moiety, which in turn allows providing a user or a person skilled in the art a molecule with reduced or increased complexity. Another reason is that full disclosure of the selected molecule can be avoided.

The determination of the first and second moiety can be performed randomly, e.g. the selected molecule is virtually divided or cut at a random molecular bond. However, as will be laid out further down, there are other ways to determine how to virtually cut the selected molecule into two moieties.

It is possible that particularly the first and/or second moiety are/is subdivided in a number of sub-moieties.

The drug score associated to the selected molecule is preferably also associated to the first and the second moiety.

An important feature of the invention is that the first moiety pharmacophore is determined preferably from a single molecule only. The first moiety pharmacophore is preferably determined from the first moiety of the selected molecule. The first moiety of the selected molecule therefore fits to the first moiety pharmacophore. It is of course also possible to determine a pharmacophore for the whole selected molecule and later subdivide the pharmacophore in the first moiety and a second moiety pharmacophore, wherein the second moiety of the selected molecule particularly fits to the second moiety pharmacophore.

As stated above, it is particularly advantageous that according to the invention the first moiety pharmacophore, but particularly also a full pharmacophore, is determined for multiple pharmacological properties, which becomes possible when using a single molecule with optimized pharmacological properties. The ideal multi pharmacological property pharmacophore or first moiety pharmacophore would be created from the optimal medical molecule or its associated first moiety respectively.

The first moiety pharmacophore may also be determined from a set of molecules or from a macromolecule, as for example a protein. Here, the first moiety pharmacophore would be an incomplete (or reduced) representation of the molecular features of the full pharmacophore.

As per definition, the first moiety pharmacophore is suited to identify structurally diverse first moieties of molecules that can bind to a biological target site.

The determined pharmacophore is preferably a two-dimensional pharmacophore, also called a 2D-pharmacophore. It is however well in the scope of the invention to generate also one-dimensional or three-dimensional pharmacophores for the purpose of identifying novel molecules for medical applications. Particularly a three-dimensional pharmacophore provides a promising perspective of graphical representations on a graphical user interface, particularly in the context of a computer game based on the method according to the invention.

A graphical user interface can be provided by or comprised in for example a monitor or electronic video goggles.

In step e) of the method, a graphical representation of a selected part of the first moiety pharmacophore is displayed on the graphical user interface.

The selected part might comprise the full first moiety pharmacophore but preferably not all molecular features of the pharmacophore are displayed at once in order to provide greater (or less specific) proposed search space for potential new molecular structures.

The graphical representations of the first moiety pharmacophore are particularly chosen such that structural or functional features similar to the first moiety of the selected molecule are comprehensible and perceivable quickly and unambiguously.

Molecular features for which a graphical representation of the first moiety pharmacophore is displayed, comprise for instance heavy atoms such as oxygen, carbon, nitrogen, atoms, terminal atoms, rings, aromatic rings, double bonds and triple bonds. Generally speaking, the molecular features mainly comprise chemical properties, especially structural ones.

This has the advantage that a person skilled in the art or any other person is enabled to not only quickly but also conveniently grasp the structural, functional and molecular properties of the first moiety pharmacophore.

In order to further reduce the complexity of the problem to design a molecule for medical applications, a starting point on the graphical user interface in relation to the graphical representation of the selected part of the first moiety pharmacophore is determined, wherein the starting point is preferably located on the atom of the second moiety, to which the first moiety of the selected molecule connects. This way the modified first moiety and the second moiety of the selected molecule always fit together to a modified molecule, as the connecting atom, i.e. the starting point, between the first modified moiety and the second moiety is non-movably displayed on the graphical user interface.

However, the starting point can be chosen randomly as well or provided by another method or a supervising instance, as long as a resulting first modified moiety fits to the second moiety.

In the following steps, a user, such as for example a person skilled in the art, is enabled to design a modified first moiety of a molecule on the graphical user interface, wherein graphical representations of molecular building blocks enable and motivate the user to quickly and comprehensibly design such optimized molecule.

The starting point in this regard serves as an origin from where the modified first moiety of the molecule is created.

As the graphical user interface displays the first moiety pharmacophore and the graphical representations of molecular building blocks that are arrangeable by the user, the user is enabled to adjust, arrange or rearrange the graphical representations of molecular building blocks such that they fit at least in parts to the represented parts of the first moiety pharmacophore.

The molecular building blocks for instance comprise (virtual) heavy atoms as listed above for the first moiety pharmacophore, and/or bonds between these atoms.

Also here, the graphical representations of the molecular building blocks are chosen such that they are particularly easy to comprehend and perceive. The graphical representations of the molecular building blocks preferably differ from the graphical representation of the first moiety pharmacophore, i.e. they are represented by different graphical elements.

As the graphical representations of the molecular building blocks are arranged starting from the starting point, arranged molecular building blocks are always interconnected, such that the associated modified first moiety of the molecule is indeed a single molecule.

A user input in order to perform the above mentioned modifications of the first moiety of the molecule is for example controlled by a computer mouse, the computer keyboard, a trackpad, a touchpad, or another human-computer interface device of a computer, such as a track ball or a pen board. The input can be translated to gestures of a pointer on the graphic display that are interpreted by a computer software as commands for shifting, turning, fixing or attaching the graphical elements to the graphical representation of the first moiety pharmacophore.

Molecular building blocks are for example either chosen by a user from a displayed selection of such molecular building blocks from the display, or selected and provided by the method from a group of molecular building blocks, comprising for example heavy atoms, such as carbon.

To each arrangement of representations of molecular building blocks a corresponding modified first moiety molecule can be determined, which leads to a modified selected molecule, when the modified first moiety and the second moiety of the selected molecule are considered together.

As the starting point is preferably chosen on the atom of the second moiety, to which the first moiety of the selected molecule connects, it is obvious how the modified first moiety and the second moiety of the molecule connect.

The new drug score in turn is determined from the molecule that consists of the second moiety and the modified first moiety, even though the second moiety is not shown on the graphical user interface.

As the drug score for the modified molecule is displayed to the user preferably in real-time or close to real-time, it enables the user to rearrange the representations of molecular building blocks on the graphical user interface such that the determined drug score increases.

In comparison to the state of the art, one advantage of the present invention is that the graphical representation of the first moiety pharmacophore and the molecular building blocks are chosen such, that they enable a user to quickly and comprehensibly grasp the task of arranging the representations of molecular building blocks with respect to the graphical representations of the pharmacophore in order to increase the displayed drug score.

Furthermore, it enables even persons that are not skilled in the art to design modified first moieties of molecules by rearranging the graphical representations of the molecular building blocks on the graphical user interface. This aspect is particularly relevant as compared to a computer that is configured to generate many variations of modified first moieties within very short time, the guesses from a human intelligence can be considered more promising, as the human intelligence discards poor or inferior solutions much quicker, particularly without even considering these solutions. A computer in contrast is bound to trial and error.

Nonetheless, as the problem of creating novel molecules for medical applications is very complex, even the guesses from a person skilled in the art are often not educated enough to design novel molecules within a reasonable period of time or a sufficient good quality.

This problem is overcome by the present invention, as it mines also the intelligence of persons not skilled in the art and thus provides access to a multitude of potential smart optimization problem solvers which in turn allows the parallelization of solvers that particularly are not bound to a common search strategy.

As each user exhibits its own optimization strategy, a variety of optimized molecules will be generated. These molecules expectedly comprise particularly unrelated conformations, structures as well as atomic compositions, which in turn increases the chance of identifying particularly viable new molecules for medical applications.

Computer algorithms in turn rely on a single, predefined search strategy or at least a limited number of search strategies, which *a priori* limits the set of potentially resulting molecules in terms of structural, conformational and atomic variety.

In a preferred embodiment of the invention the steps g) to k) are repeated until the drug score of the modified molecule is higher than the drug score of the selected molecule.

An increased drug score indicates a potentially optimized modified molecule with respect to the initially selected molecule. Thus, the drug score is preferably an indicator that reflects the pharmacological properties of a molecule, wherein said indicator preferably is configured to distinguish molecules that have better pharmacological properties from molecules that have inferior pharmacological properties.

In another embodiment of the invention step g) is performed by a collective intelligence, particularly by a plurality of users, wherein the method is executed, particularly simultaneously, on a plurality of instances.

The collective intelligence comprises for example a plurality of computer programs that are arranging or rearranging the representations of molecular building blocks. Preferably, as laid out above, the collective intelligence originates from a plurality of independently arranging and rearranging users or persons. The approach of using a collective intelligence is particularly advantageous as almost nothing is known about the chemical space and in which part the optimized molecule can be found. It is not clear which search strategy through this chemical space will yield the best results within a reasonable time span. The chemical space in this context comprises all possible molecules, with an estimated size of around $10^{60}$ for molecules with a molecular weight of up to 500g/mol.

A collective intelligence is therefore advantageous as no such *a priori* knowledge is required.

It is particularly advantageous to execute the method according to the invention multiple times and particularly simultaneously or quasi-simultaneously on a plurality of instances, such as computers in order to grant a parallelized approach to the problem of designing optimized molecules.

According to a preferred embodiment of the invention on each instance, such as a computer, a modified molecule is determined, such that a plurality of modified molecules with increased drug score is determined.

This aspect of the invention is particularly advantageous as from the plurality of modified molecules, specific molecules can be selected that are assigned for further optimization.

According to a preferred embodiment of the invention a physics simulation engine is provided, wherein said engine adjusts the position of the graphical representations of the molecular building blocks according to their associated molecular properties on the graphical user interface, wherein the associated molecular properties particularly comprise at least one of:

- repulsive and attracting forces between the representations of the molecular building blocks,
- an exclusion size, such as for example atomic radii or bonds, of the represented molecular building blocks,
- a length, such as for example atomic bonds, of the represented molecular building blocks.

The physics simulation engine is a computer program that is configured to particularly minimize the energy of an associated molecule for example by force field calculations or other well-known methods. Furthermore the physics simulation engine is particularly configured to allow only certain arrangements of atoms with respect to each other. For example the adoptable angle enclosed by two atomic bonds might be fixed to a limited number of values.

Furthermore the physics engine is particularly configured to automatically recognize predefined associated molecular structures, for example an arrangement that is a ring structure, which may be changed by the user, to for example an aromatic ring. Such changes of predefined molecular structures, commonly encountered in molecules, are then suggested to a user arranging the representations of the molecular building blocks on the graphical user interface.

The physics simulation engine makes time consuming manual adjustment unnecessary. Particularly its automatic recognition of predefined molecular structures is advantageous as the design-process is accelerated.

Also, the physics simulation engine limits the possible solutions which fit to the molecular features of the pharmacophore by only allowing appropriate geometries between atoms and atomic bonds, accelerating the design-process even more.

According to a preferred embodiment of the invention, the drug score is determined by the steps of:

- providing a multi-objective function that is configured to process an electronic, particularly a computer-readable representation of a molecule and to determine the drug score for said molecule, wherein the multi-objective function comprises a plurality of objective functions, wherein each objective function is configured to process the electronic representation of the molecule and to determine the strength of a specific pharmacological property of the molecule, and wherein said multi-objective function is particularly a combination or a function of the plurality of objective functions,

- and when these objective functions are weighted amongst each other so that the multi-objective function distinguishes (ranks) the medical molecules from molecules with not suitable pharmacological properties,

- determining the drug score of the modified molecule by evaluating the multi-objective function for the modified molecule.

According to another embodiment of the invention, the objective functions are particularly supervised learning models, wherein the learning model is particularly a Quantitative Structure Activity Relationship (QSAR) or a Quantitative Structure Property Relationship (QSPR) model.

QSAR and QSPR models for example are created from a set of molecules with experimentally determined and known outcomes; for the present invention the experimentally determined and known outcomes are the pharmacological properties, which may both be pharmacodynamic or pharmacokinetic properties. Molecules with pharmacological properties may be obtained from public sources as for example ChEMBL (https://www.ebi.ac.uk/chembl/).

To create a QSAR or QSPR model, the chemical, structural and/or physical properties, as for example the detailed structural composition or the electronic characteristics, respectively, of each molecule belonging to a set of molecules have to be determined. Different sets of chemical, structural and/or physical properties of each molecule are then related through different mathematical functions to an outcome of each molecule, here a pharmacological property. Different combinations of chemical, structural and/or physical properties with different mathematical functions result in worse or better QSAR or QSPR models, which can predict the pharmacological property for a new molecule on basis of its related chemical, structural and/or physical properties.

Mathematical functions for relating the chemical, structural and/or physical properties of each molecule to the outcome, here principally the pharmacological property, comprise classification algorithms or regression algorithms, such as for example Naive Bayes classifier or Partial Least Square (PLS) regression respectively. Suitable models and predictions are for example achieved by using best practice QSAR and QSPR modeling techniques as published elsewhere [2]. The quality of QSAR or QSPR models are evaluated by internal and external accuracy and reliability filters as published elsewhere [3], which allow to for example estimate the error in the prediction of the pharmacological property. QSAR or QSPR models can only be applied to molecules with certain chemical, structural and/or physical properties, which can be estimated by the so called applicability domain.

Applicability domain estimations are preferably applied with the best QSAR or QSPR model in the objective function to minimize prediction errors.

Objective functions are modelled for at least one of the following pharmacological properties, which may both be pharmacodynamic or pharmacokinetic properties:

- therapeutic effect, wherein the therapeutic effect refers particularly to the interference of the molecule with the or more biological targets, often a protein or a plurality of proteins, which results in an improvement of a disease.

- side effects, particularly those related to the therapeutic effect, which result in unwanted responses particularly of the human body, as for example a decrease in the therapeutic effect through binding of similar biological targets or vomiting, dizziness and/or psychological problems through binding to neuronal targets,

-   toxicity, particularly cardiovascular toxicity or hepatoxicity, as for example the interaction with the ion-channel protein hERG or toxic metabolites created by cytochrome enzymes, respectively, which may cause short and also long term health problems in the patient and/or

-   pharmacokinetics, as Absorption, Distribution, Metabolism and Excretion (ADME), referring for example to the uptake of the molecule into the blood system through for example the digestive track, the disposition of the pharmacological molecule to the effector site and therefore the biological target, the break-down of the pharmacological molecule to metabolites over time and the removal of all metabolites. Summarizing, a good disposition of a medical molecule in the body allows the molecule to act effectively and efficiently.

Alternatively, the objective functions are determined from a scoring function based on a force field, from an empirical approach, a semi-empirical approach or a knowledge-based approach.

According to another embodiment of the invention, the first moiety of the selected molecule comprises at least 1 heavy atom of the selected molecule, preferably all heavy atoms, particularly more than 75% of the heavy atoms of the selected molecule, most particularly more than 25% of the heavy atoms of the selected molecule.

Heavy atoms are considered all atoms except hydrogen.

According to another embodiment of the invention, the selected part of the first moiety's pharmacophore comprises more than 10% of the molecular features of the first moiety pharmacophore, particularly more than 50% of the molecular features of the first moiety pharmacophore, more particularly more than 85% of the molecular features of the first moiety pharmacophore.

The selected part of the first moiety pharmacophore controls the degree of information that is displayed to a user when arranging the representation of molecular building blocks on the graphical user interface. By controlling the degree of information about the first moiety pharmacophore, it is possible to extend the proposed search space for a user, such that high-scoring modified first moieties of the molecule are potentially structurally very different.

According to another embodiment of the invention, the selected part of the first moiety's pharmacophore is increased, when the drug score of the modified molecule is below the drug score of the selected molecule after particularly repeatedly executing steps g) to k).

If the selected part of the first moiety pharmacophore is small, than the proposed search space for modified first moieties increases, and it might be too difficult to find modified moieties with an increased drug score. Therefore, it is advantageous to increase the selected part of the first moiety pharmacophore in order to reduce the search space and to provide the user with hints, where a potentially optimized modified first moiety can be found.

According to another embodiment of the invention, additional graphical representations of pharmacophore features are displayed on the graphical user interface.

These additional molecular features of the pharmacophore can be displayed in order to give a user an incentive to explore a certain part of the search space more thoroughly, even though the first moiety pharmacophore does not contain these additional molecular features. The advantage of displaying these additional molecular features is that a potentially greater variety of modified first moieties of molecules is generated.

It is understood that a molecular feature of the pharmacophore may end up being interchanged for an additional molecular feature. This happens if in the same position, where a molecular feature of the pharmacophore was eliminated, a new but different molecular feature is placed.

According to another aspect of the invention, the plurality of modified molecules is further optimized performing the following steps:

-   determining a plurality of Pareto fronts of the plurality of modified molecules, wherein particularly the 10th-best to first-best Pareto front is determined and wherein the Pareto fronts are determined with regard to two, three or four combinations comprised of e.g. all the objective functions,

-   ranking the molecules comprised by each Pareto front either separately for each Pareto front or jointly according to the strength of one of the specific pharmacological properties, for example by the strength of the therapeutic effect,

-   executing at least the steps a) to k) again, wherein the selected molecule is one of the modified molecules that are comprised by the highest ranked modified molecules, particularly the highest ranked molecule.

Alternatively it is also possible to either select a new selected molecule manually through human knowledge or selecting a new selected molecule with a desired drug score or selecting a new selected molecule by thresholds of a specific number of endpoints, especially pharmacological ones. The new selected molecule can subsequently be chosen for another iteration with the method according to the invention.

The Pareto front, also called Pareto frontier or first-best Pareto front, is the set of modified molecules that particularly comprise favorable combinations of pharmacological properties determined from the objective functions, wherein it is not possible amongst the set of modified molecules to increase the strength of any of the pharmacological properties without reducing the strength of any other pharmacological property. Therefore the Pareto front is the set of modified molecules that are Pareto efficient with regard to the strength of the pharmacological properties determined from the objective functions.

It is obvious that the objective for the pharmacological property "side effects" is to reduce this pharmacological property. Therefore the strength of a pharmacological property has to be chosen appropriately, e.g. by assigning the inverse value for the pharmacological property "side effect" as the strength.

The second-best Pareto front is the set of modified molecules that comprises pharmacological properties with strengths such that one of the pharmacological properties can be increased one time without decreasing at least one of the other pharmacological properties of the modified molecule. In other words, the second Pareto front would be the first Pareto front when taking out/not considering the set of modified molecules making up the first Pareto front.

Analogously it is possible to define the 3rd-best, 4th -best and higher-order Pareto fronts.

The highest ranked molecules preferably consist of the 10 highest ranked molecules. As it is potentially more difficult to increase the drug score of a modified molecule that is already comparably high ranked, it is advantageous to also consider molecules that are ranked lower than the highest ranked molecules. For the same reason it is advantageous to consider molecules comprised by the higher-order Pareto frontiers.

As these molecules serve as a new selected molecule, it is advantageous to maintain a variety of molecules in order to maintain a structural variety.

According to another embodiment of the invention, the method according to the invention further comprises the steps of:

- determining for at least one of the highest ranked molecules a plurality of moieties, wherein particularly the drug score from the respective molecule is assigned to each of the moieties of the plurality of moieties of the respective molecule,

- determining a molecular complexity for each moiety of the plurality of moieties of the respective modified molecule,

- particularly assigning the molecule with the moiety associated to the highest complexity as the selected molecule and the respective moiety to the first moiety of the selected molecule.

Alternatively to assigning the moiety with the highest complexity to the first moiety it is also advantageous to assign a moiety with a lower complexity to the first moiety of the selected molecule. As the method particularly aims to enable a variety of particularly differently skilled person to perform optimization on molecules, it is possible to provide each person depending on its skill and experience a different moiety comprising a complexity according to their experience. The experience can for example be determined by previous runs of the method, where it is evaluated how skilled the person is in designing new molecules.

This can be advantageously done in a computer game environment, where the method is incorporated as a computer or an online-computer game. Each time a person has increased the drug score of a molecule, a new level within the computer game is achieved wherein in the new level particularly the complexity of the first moiety is increased. Also, it is possible to provide the experienced gamer with molecules from the best Pareto fronts, wherein an experienced gamer is a gamer who has played through a predefined number of levels.

The problem according to the invention is also solved by a computer program, wherein the computer program comprises computer executable code that prompts a computer to execute the method according to the invention, when the computer program is executed on a computer.

Furthermore the problem according to the invention is solved by a micro-processor comprising the computer program according to the invention.

Another aspect of the invention relates to a system, particularly a computerized system, for designing a molecule for medical applications by optimization of an associated drug score of the molecule, the system comprising at least one client and a server operationally connected to the at least one client, wherein the server comprises:

    a storage unit for storing a computer-readable representation of selected molecules, a drug score and the individual parts of the drug score associated to each of the selected molecule;

    a processor operationally connected to the storage unit and configured to:

    - select a molecule for display on a graphical user interface of the at least one client,

    - prompt to display the graphical representation of a selected part of a first moiety pharmacophore on the graphical user interface of the client,

    - prompt to display the graphical representations of the molecular building blocks on the graphical user interface of the at least one client,

    - receive user input from the at least one client, wherein the user input is suited to instruct the client to rearrange the graphical representations of molecular building blocks on the display and save it on the server, wherein the input is provided by a user via an input unit such as a computer mouse, a computer keyboard, a touchscreen, a voice control unit, an accelerometer and/or any combinations thereof; and

wherein the client comprises:

- a processing unit configured to translate the user input into suitable commands for the server and to process data received from the server,

- a display unit configured to display the graphical user interface; and

- an input unit configured to obtain input data from the user, wherein the input data is configured to prompt the processor to arrange the graphical representations of the molecular building blocks.

Further, the client or server or both can be configured to perform the steps of

- saving the arranged molecular building blocks on the storage of the client until sending it to the server,

- calculating the pharmacological properties including the drug score of the arranged molecular building blocks, or

- saving the arranged molecular building blocks and the calculated pharmacological properties including the drug score temporarily on the storage of the client until sending it to the server.

One realization of the system is a network-based optimization system, or an online optimization system, wherein the method according to the invention is performed as a computer game on the clients. The clients might be personal computers, tablets, or other computer-like mobile devices, such as smartphones and smart-watches.

The server in turn is for example a computer that is capable of processing and executing the method or the computer program according to the invention, particularly simultaneously on a plurality of clients. This approach is particularly advantageous as it enables the mining of many peoples intelligences that take part in the optimization of the selected molecule or a plurality of selected molecule, such that the optimizations procedure is likely to yield optimized molecules.

Further, the invention can be implemented as a computer game system, comprising various components that are configured to execute the computer game on a client and/or a computer.

According to one embodiment of the invention a video game system comprises a control processor for playing a video game for designing a molecule for medical applications by optimization of an associated drug score of a molecule, wherein the video game system comprises means for estimating the associated drug score of a selected or modified molecule, wherein graphical representations of molecular building blocks are arrangeable by a game player such that the molecular building blocks can be interconnected and form a graphical representation of a modified first moiety of a molecule, wherein the game player improves the resulting drug score by modifying the modified first moiety of the molecule.

According to another embodiment of the invention the video game system includes a physics simulation engine, wherein said engine adjusts the position of the graphical representations of the molecular building blocks, preferably without the necessity of interaction of a game player, according to their associated molecular properties on the graphical user interface, wherein the associated molecular properties particularly comprise at least one of:

- repulsive and attracting forces between the representations of the molecular building blocks,

- an exclusion size of the represented molecular building blocks,

- a length of the represented molecular building blocks.

The invention can further be realized by a video game for designing a molecule for medical applications by optimization of an associated drug score of a molecule, wherein a game player of the video game is performing step g) of the method according to the invention and wherein the video game is configured to execute or provide the steps a) to k) of the method according to the invention.

According to another embodiment of the invention the video game is configured to estimate a drug score of a modified molecule, wherein a first moiety of the modified molecule is modified by a game player during game play in order to increase its associated drug score, wherein each time a game player increases the associated drug score of the molecule sufficiently, the game player is advanced to a next level of game play, where another first moiety of a selected molecule is represented to the game player. Further features and advantages of the invention shall be described by means of a detailed description of embodiments with reference to the Figures, wherein

Fig. 1    a flow chart with a graphical user interface;

Fig. 2    a flow chart with the level selection;

Fig. 3    a graphical user interface according to the invention; and

Fig. 4    an infrastructure of the computerized system according to the invention;

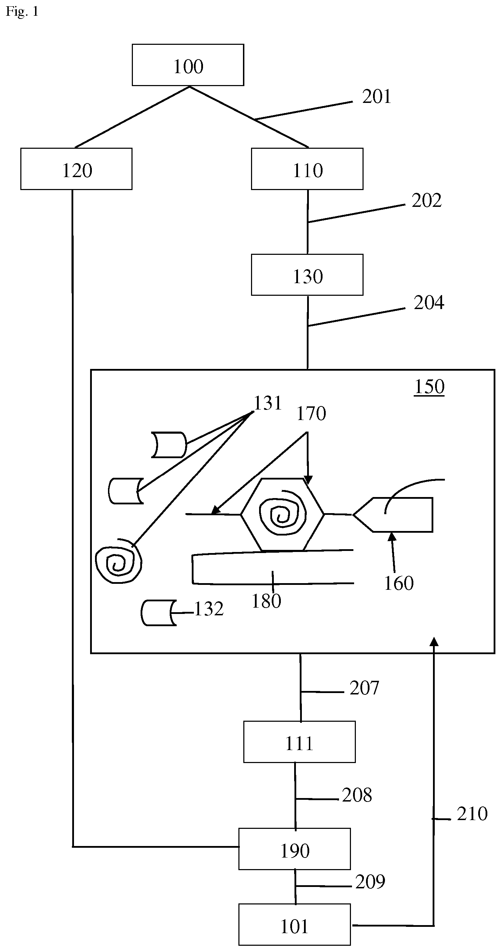

Fig. 1 shows a flowchart diagram for the method according to the invention applied to a selected molecule 100. In a first step a computer-readable representation of the selected molecule 100 is provided, wherein a first moiety 110 of said molecule 100 is determined 201.

On a graphical user interface 150 (GUI) a graphical representation of a first moiety pharmacophore 130 is displayed, wherein preferably only a selected part 131 of the first moiety pharmacophore 130 is displayed 204. The representation of the first moiety pharmacophore 130 is not rearrangable on the GUI 150 by a user.

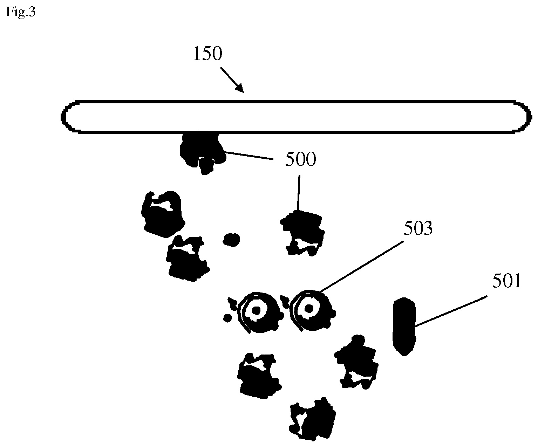

In this example the method according to the invention is comprised in a computer game, wherein the GUI 150 comprises a background image, such as for example a cartoon of an ocean or a lake viewed from above. On the background image a graphical representation of the selected parts 131 of the first moiety pharmacophore 130 is given by islands 500 (see also Fig. 3), representing atoms, vortices 503, representing molecular ring structures, and a starting point 160, represented by a boat 501 (see also Fig. 3). The islands 500 might comprise different colors or features, such that the islands represent different kind of atoms, e.g. carbon in red or nitrogen in blue for example.

A user playing the computer game is now arranging and rearranging molecular building blocks 170, represented as for example bases with different end features, wherein depending on the color and/or shape of the bases only certain bases are interconnectable or connectable through forces to the islands 500. The different bases correspond to bonds between different atoms and the length of the base corresponds to the bond between the atoms. Also, each base comprises an atom, which might not be graphically represented.

Therefore, as the various molecular building blocks 170 are all connected to each other (starting from the starting point 160), a virtual molecule is formed. This molecule corresponds to a first modified moiety 180 of the selected molecule 100. This modified first moiety 180 is connected to a (invisible) second moiety 120 of the selected molecule 100, wherein the resulting modified molecule 190 consists of first modified moiety 180 and the second moiety 120.

Each time the user adds, removes or rearranges a molecular building block 170, a drug score 101 for the modified molecule 190 is re-evaluated and displayed 210 on the GUI 150. In some particular situations, a previously attained drug score may be shown, especially the highest drug score achieved during a level.

This way, the user is provided with a feedback about the newly created molecule. The goal for a given selected molecule 100 comprising a certain drug score 101 is to create a new modified molecule 190 with a modified first moiety 180, such that the drug score 101 of the modified molecule 190 is higher than the drug score

101 of the selected molecule 100.

Thus, the user is asked to reach a certain drug score 101 in the computer game by adding, removing and / or rearranging molecular building blocks 170 to a new modified first moiety 180 of the molecule 190.

Once the user reached or surpassed the targeted drug score 101, the user has finished a level of the computer game and can proceed to a next level that preferably starts with a different first moiety 110a, 110b, 110c particularly from a different selected molecule, and wherein the first moiety pharmacophore 130 is more complex than the pharmacophore from the previous level.

Physics and chemistry simulation on the GUI:

An important feature of the method according to the invention is that a physics simulation engine is provided, particularly for the following tasks:

-    the physical simulation engine provides predefined physical laws to the molecular building blocks 170, and

-    the physics simulation engine recognizes common molecular structures and is configured to suggest such a common molecular structure, when molecular building blocks are arranged similarly to such a structure.

The physics simulation engine is configured to also provide some chemistry simulations tasks, such as the common molecular structure recognition.

The physical laws provided to the molecular building blocks 170 comprise for example some repulsive or attractive forces between molecular building blocks 170. Also, size exclusion of the molecular building blocks 170 is recognized. For example, it is not possible for the user to arrange two atoms in the same place or in an overlapping manner on the GUI 150.

The recognition of the physics simulation engine of common molecular structures, as for example aromatic rings, facilitates a quick and convenient assembly of modified first moieties 110a, 110b, 110c. Thus, if for example a user arranges molecules in a manner that they could create an aromatic ring, the physics simulation engine suggests to change the molecular building blocks to an aromatic ring, and if the user agrees then the physics simulation engine changes the molecular building blocks to an aromatic ring.

Furthermore the physics simulation engine allows only certain angles between the molecular building blocks, such that creating modified first moieties is facilitated and comparably easy, as most molecules exhibit a limited number of angles between their atoms.

The physics simulation engine for example can be conveniently included using a physics simulator called box2d, from box2d.org. This engine simulates the physical world, offering the possibility to define parameters like mass, time, distance and acceleration to simulate physical properties like velocity and forces like repulsion and attraction of the molecular building blocks.

During execution of the method and particularly during game play, the molecular building blocks 170 are optimized in the millisecond range regarding the underlying physics. A user might virtually pull on a molecular building block 170 to rearrange the molecular building blocks 170. Upon relaxation (no more pulling) the molecular building blocks 170 adjust to the physical laws provided by the physics simulation engine

One way to facilitate the correct behavior of the molecular building blocks 170, particularly the atoms of the modified first moiety, is described in the following:

a) all atoms of the molecular building blocks are connected to three other atoms, either visible atoms or to so-called ghost-atoms (so the angle is always 120 degrees amongst atoms) that are hidden to the user. Atoms the user placed on the GUI 150 (visible atoms) are considered in the determination of the drug score. The hidden ghost-atoms, which the user did not place, are not displayed on the GUI 150 and only serve to assure an appropriate geometry of the molecule on the GUI 150.

b) if an atom has a triple bond, one of the ghost atoms is deleted, causing the molecular structure to be linear and have 180 degree bonds between 3 atoms.

c) different types of atoms exist. The user is limited to the number of bonds the specific atom can make (for example, oxygen can only have two bonds and can therefore be connected to one or two). It should be noted that in the case of carbon, which may bind four atoms, the angle between the atoms automatically changes to 90 degrees, when four atoms are attached to it.

d) upon ring creation:

1) all bonds (real and ghost-atoms) which do not participate in the cycle are flipped out of the ring, putting the ring in the right conformation.

2) ring closure is only allowed between two atoms with a predefined maximum distance.

3) ring closure is only allowed between two atoms if less than three visible bonds are crossed

Selected and additional pharmacophore parts 132:

During execution of the method only a selected part 131 of the first moiety pharmacophore 130 is displayed on the GUI 150, in order to provide a big enough search space for the user.

The selected part 131 shown on the GUI 150 can be increased during execution of the method if, for example, the drug score 101 is not increasing after a predefined number of rearranging attempts of the molecular building blocks 170, or if the user requests so. The selected part 131 of the first moiety pharmacophore 130 also depends on the computer game level.

In order to increase the search space for molecules, it is also possible to display additional parts 132 on the GUI 150, wherein said additional parts 132 are molecular features of the pharmacophore, i.e. they are represented in the same graphical manner as the other molecular features of the pharmacophore on the GUI 150, even though these additional parts 132 are not part of the pharmacophore. Still, these additional parts may be placed in the same position where a molecular feature of the pharmacophore was eliminated. These additional parts 132 provide an incentive to a user to arrange the molecular building blocks 170 such that they connect these additional parts 132 as well.

Level selection:

In order to build a computer game that comprises the method according to the invention, various levels of the computer game have to be generated, wherein each level preferably is slightly more difficult than the preceding level.

In case of designing a video game, the difficulty does not always have to rise from level to level. Easier levels may be generated at higher levels to allow the gamer to win the level faster and motivate the gamer.

In order to achieve this, the method according to the invention includes a processor that is configured to automatically create new levels based on previously found modified molecules 190a, 190b, 190c.

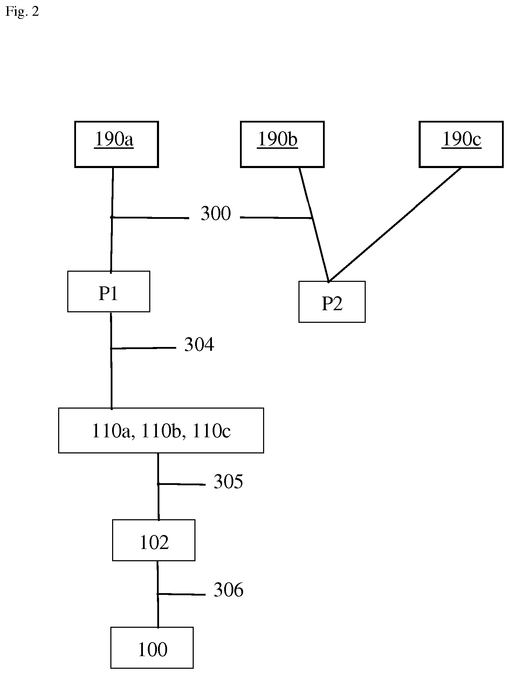

In Fig. 2 it is shown how the level generation can be facilitated:

In a first step, the Pareto front P1 of all previously modified molecules 190a, 190b, 190c is estimated 300, with regard to the strength of their pharmacological properties.

It is also possible to determine the second-best and other higher-order Pareto fronts P2, which comprise modified molecules 190b, 190c that generally have a higher drug score 101 than the initially selected molecule 100 but a lower drug score 101 than the modified molecules 190a from the (first) best Pareto front P1.

These Pareto molecules are than ranked according to the strength of a pharmacological property, mostly the "therapeutic effect".

For each of the preferably ten best molecules from this ranking, a plurality of first moieties 110a, 110b, 110c is determined 304 by a so-called cutting algorithm that is explained further below. Each first moiety 110a, 110b, 110c has the drug score 101 from the respective molecule 190a assigned to it. The moieties 110a, 110b, 110c are filtered such that only moieties bigger than 1 atom and smaller than a predefined number of atoms, for example 30 or 50, are processed further. In a next step the complexity 102 of each moiety 110a, 110b, 110c is determined. For a new level a first moiety pharmacophore 130 is created from a first moiety with an appropriate complexity 102, i.e. a low level will be provided with a first moiety pharmacophore 130 with low complexity whereas for a higher level a first moiety pharmacophore 130 with a comparably high complexity 102 will be presented on the GUI 150.

Complexity evaluation.

The complexity 102 of a computer game level can be described by the molecular complexity 102 of the first moiety 110a, 110b, 110c from the cutting algorithm.

To estimate 305 the molecular complexity 102, the number of unique components of the first moiety 110a, 110b, 110c is determined. In the present case, a component represents one atom of the first moiety. The components and the component properties can be determined by the state-of-the-art method "extended connectivity fingerprints" (ECFP-like), A.K.A. circular fingerprints.

In order to determine the unique components, for each heavy atom of the first moiety 110a, 110b, 110c a code (also called fingerprint) is determined and assigned to the respective atom.

The code comprises and represents properties like: element type, number of connections, number of attached implicit hydrogen atoms, atom charge, atom mass, belonging to a ring, etc., also of atoms connected through multiple bonds to the respective atom.

If two atoms of the first moiety 110a, 110b, 110c have the same properties, then they have the same code in case of ECFP0.

Finally, the number of unique codes that corresponds to the number of unique components is determined. In the combination of the unique codes, some specific codes may also be weighted differently than 1, giving more or less importance to such specific codes. The higher this number is, the higher the complexity 102 of the corresponding first moiety 110, 110a, 110b, 110c is.

Cutting algorithm:

The following protocol can be applied in order to generate a first moiety 110, 110a, 110b, 110c of a molecule.

- Providing a selected molecule,

- For each bond B of the selected molecule connecting an atom A1 and an atom A2, that does mainly not belong to a cycle

- Cutting B and generate a first moiety comprising atom A1,

- Validating if the obtained first moiety upon cutting consist of at least 1 and at the most all atoms of the selected molecule, more particularly between 25% to 75% of heavy atoms of the selected molecule. If this is the case,

- Adding the bond B to A1 and assigning atom A2 as the starting point.

Drug score determination:

The drug score 101 of a molecule is determined by QSAR / QSPR modeling.

A comprehensive overview over the QSAR modeling can be found in [2] and [3].

From the QSAR model, pharmacological properties of a molecule can be determined. These pharmacological properties, which may both be pharmacodynamic or pharmacokinetic properties, can be expressed as an objective function and output a value related to for example:

- a therapeutic effect,

- a toxicity,

- a hepatoxicity,

- a water solubility,

- an albumin binding,

- a cytochrome p450 binding,

- an off-target binding.

Therefore it is straight forward to estimate a multi-objective function that summarizes all the estimated pharmacological properties in a single value that is called the drug score 101. The multi-objective function is for example given by a linear combination of the following objective functions Fi:

F1 = therapeutic effect

F2 = side effects

F3 = toxicity

F4 = ADME

The drug score 101 (DS) therefore can be calculated by, with y1, y2, y3 and y4 being real-valued coefficients.

$$DS = y1*F1 + y2*F2 + y3*F3 + y4*F4.$$

In Fig. 3 a GUI 150 is shown that shows the molecular building blocks as islands 500, as well as curls 503 that are thought to motivate a user to build certain structures around them in order to contain them, wherein the curls 503 are suggesting a molecular ring structure to be built. The starting point is depicted as a boat 501 on the ocean.

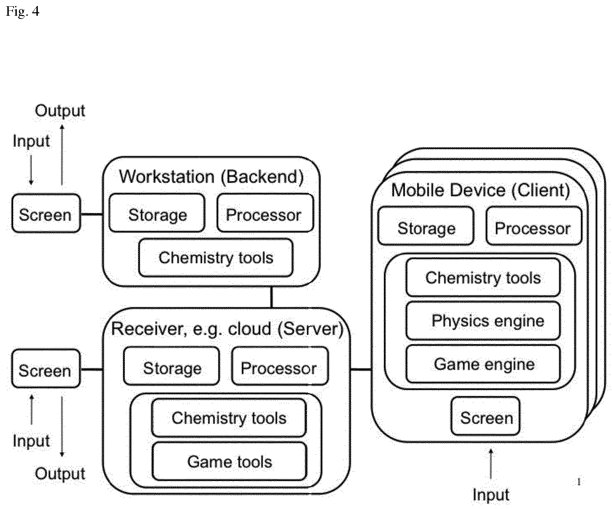

Fig. 4 shows an exemplary infrastructure of the computerized system according to the invention wherein the computerized system is configured to execute the method according to the invention.

The system comprises at least one Mobile Device (Client) that is configured to perform the tasks of adjusting the position of the graphical representations of the molecular building blocks 170 according to their associated molecular properties on the graphical user interface 150, wherein the associated molecular properties particularly comprise for example repulsive and attracting forces between the representations of the molecular building blocks, an exclusion size of the represented molecular building blocks, a length of the represented molecular building blocks. These tasks are for example executed with the aid of a physics engine. The Client is further configured to perform the evaluation of the drug score 101, particularly using the Chemistry tools. Furthermore the Client is configured to execute various tasks (as for example disclosed in claims 2, 3, 4, 10 and 11 as well as the steps d) to k) of claim 1) using a Game engine.

The Client is connected (connection in the present context refers to the ability of the devices to electronically exchange data) to the Receiver that is preferably a cloud, e.g. a plurality of servers, wherein the cloud is configured to collect and store data in the Storage from Client and Backend. The Receiver is for example configured to execute claims 8, 9, 13, 14 and particularly steps a), b), c) and f) of claim 1 using the help of the Game tools. The Receiver is also configured to perform the tasks of claims 6, 7 and partially claim12 with the help of the Chemistry tools. The Receiver can be controlled and modified through the Screen with the Output and Input.

The Workstation (Backend) is connected to the Receiver and is configured to perform the tasks of extracting molecules and data from the Storage of the Receiver. The extracted molecules and also new molecules, which are created by the Backend, are further analyzed with Chemistry tools before sending all molecules and data back to the Storage of the Receiver.

User input is provided either via the Screen of the Client, by specifying the arranging and rearranging and the Screen of the Backend.

A plurality of Clients can be addressed via the cloud. Thus the method according to the invention can be performed simultaneously on multiple Clients, such that an optimization of a molecule or a plurality of molecules and their associated moieties is facilitated by the plurality of Clients. The Clients comprise a Screen that is configured to display the (representations of the) first moieties and other associated molecular building blocks.

In the context of embodiments of the present disclosure, by way of example and without limiting, terms such as 'operating' or 'executing' imply also capabilities, such as 'operable' or 'executable', respectively. Conjugated terms such as, by way of example, 'a thing property' implies a property of the thing, unless otherwise clearly evident from the context thereof.

The terms 'processor' or 'computer', or system thereof, are used herein as ordinary context of the art, such as a general purpose processor or a micro-processor, RISC processor, or DSP, possibly comprising additional elements such as memory or communication ports. Optionally or additionally, the terms 'processor' or 'computer' or derivatives thereof denote an apparatus that is capable of carrying out a provided or an incorporated program and/or is capable of controlling and/or accessing data storage apparatus and/or other apparatus such as input and output ports. The terms 'processor' or 'computer' denote also a plurality of processors or computers connected, and/or linked and/or otherwise communicating, possibly sharing one or more other resources such as a memory.

The terms 'software', 'program', 'software procedure' or 'procedure' or 'software code' or 'code' or 'application' may be used interchangeably according to the context thereof, and denote one or more instructions or directives or circuitry for performing a sequence of operations that generally represent an algorithm and/or other process or method. The program is stored in or on a medium such as RAM, ROM, or disk, or embedded in a circuitry accessible and executable by an apparatus such as a processor or other circuitry.

The processor and program may constitute the same apparatus, at least partially, such as an array of electronic gates, such as FPGA or ASIC, designed to perform a programmed sequence of operations, optionally comprising or linked with a processor or other circuitry.

The term computerized apparatus or a computerized system or a similar term denotes an apparatus comprising one or more processors operable or operating according to one or more programs.

As used herein, without limiting, a module represents a part of a system, such as a part of a program operating or interacting with one or more other parts on the same unit or on a different unit, or an electronic component or assembly for interacting with one or more other components.

As used herein, without limiting, a process represents a collection of operations for achieving a certain objective or an outcome.

As used herein, the terms 'server' or 'client' or 'backend' denotes a computerized apparatus providing data and/or operational service or services to one or more other apparatuses.

The term 'configuring' and/or 'adapting' for an objective, or a variation thereof, implies using at least a software and/or electronic circuit and/or auxiliary apparatus designed and/or implemented and/or operable or operative to achieve the objective.

A device storing and/or comprising a program and/or data constitutes an article of manufacture. Unless otherwise specified, the program and/or data are stored in or on a non-transitory medium.

The term 'operationally connected' denotes a computerized network connection or communication via a network, e.g. a cellular network, wireless network such as radio, Bluetooth or WiFi, a wired network such a Local Area Network (LAN) or Wide Area Network (WAN), as well as a connection via the internet.

The flowchart and block diagrams illustrate architecture, functionality or an operation of possible implementations of systems, methods and computer program products according to various embodiments of the present disclosed subject matter. In this regard, each block in the flowchart or block diagrams may represent a module, segment, or portion of program code, which comprises one or more executable instructions for implementing the specified logical function(s). It should also be noted that, in some alternative implementations, illustrated or described operations may occur in a different order or in combination or as concurrent operations instead of sequential operations to achieve the same or equivalent effect.

List of selected reference numerals:

200     providing a computer-readable representation of a selected molecule

201     determining a first moiety and a second moiety of the selected molecule

202     determining a first moiety pharmacophore

204     displaying a graphical representation of a selected part of the first moiety pharmacophore on the graphical user interface

207     assigning the graphical representation of the modified first moiety of the molecule to a modified first moiety of the selected molecule,

208     determining a modified molecule consisting of the modified first moiety and the second moiety of the selected molecule,

209     estimating the associated drug score for the modified molecule,

210     disclosing, particularly displaying on a display unit, the associated drug score of the modified molecule

300     determining a plurality of Pareto fronts of the plurality of modified molecules

304     determining for at least one of the highest ranked molecules a plurality of moieties

305     determining a molecular complexity

306     assigning the molecule with the moiety associated to the most appropriate complexity as the selected molecule

References:

[1] Bohacek, Mcmartin, Guida; "The Art and Practice of Structure-Based Drug Design A Molecular Modeling Perspective ", Medicinal research reviews 1996,

[2] Tropsha A., "Best Practices for QSAR Model Development, Validation, and Explotation", Mol. Inf. 2010, 29, 476-488

[3] Eriksson L. et al., "Methods for Reliability and Uncertainty Assessment and for Applicability Evaluations of Classification- and Regression-Based QSARs", EHP 2003, Volume 111, No. 10, 1361-1375.

**Claims**

1.   Method for designing a molecule for medical applications by optimization of an associated drug score (101) of the molecule, wherein the method is a computer-implemented method performed by a computerized device comprising a processor, comprising the steps of:

> a) providing a computer-readable representation of a selected molecule (100) and a drug score (101) associated to the selected molecule (100),
> b) determining (201) a first moiety (110) of the selected molecule (100) and a second moiety (120) of the selected molecule (100), wherein the selected molecule (100) consists of the first moiety (110) and second moiety (120),
> c) determining (202) a first moiety pharmacophore (130), wherein the first moiety (110) of the selected molecule (100) fits to the first moiety pharmacophore (130), and wherein the first moiety pharmacophore is determined from a single molecule only,
> d) providing a graphical user interface (150),
> e) displaying (204) a graphical representation of a selected part (131) of the first moiety pharmacophore (130) on the graphical user interface (150),

f) determining a starting point (160) on the graphical user interface (150) in relation to the graphical representation of the selected part (131) of the first moiety pharmacophore (130),

g) from the starting point (160), arranging graphical representations of molecular building blocks (170) on the graphical user interface (150), wherein the graphical representations of the molecular building blocks (170) are interconnected and form a graphical representation of a modified first moiety of a molecule (180),

h) assigning (207) the graphical representation of the modified first moiety of the molecule (180) to a modified first moiety (111) of the selected molecule (100),

i) determining (208) a modified molecule (190) consisting of the modified first moiety (111) and the second moiety (120) of the selected molecule (100),

j) estimating (209) the associated drug score (101) for the modified molecule (190),

k) disclosing (210) the associated drug score (101) of the modified molecule (190).

2. Method according to claim 1, wherein the steps g) to k) are repeated until the drug score of the modified molecule (190) is higher than the drug score (101) of the selected molecule (100).

3. Method according to claim 1 or 2, wherein step g) is performed by a collective intelligence, particularly by a plurality of users, wherein the method is executed, particularly simultaneously, on a plurality of platforms, particularly computers.

4. Method according to claim 3, wherein on each platform a modified molecule (190) is determined such that a plurality of modified molecules (190a, 190b, 190c) with increased drug score (101) is determined.

5. Method according to one of the preceding claims, wherein a physics simulation engine is provided, wherein said engine adjusts the position of the graphical representations of the molecular building blocks (170), preferably without the necessity of any human interaction, according to their associated molecular properties on the graphical user interface (150), wherein the associated molecular properties particularly comprise at least one of:

- repulsive and attracting forces between the representations of the molecular building blocks,
- an exclusion size of the represented molecular building blocks,

- a length of the represented molecular building blocks.

6. Method according to one of the preceding claims, wherein the drug score (101) is determined by the steps of:

- providing a multi-objective function that is configured to process an electronic representation of a molecule and to determine the drug score for said molecule, wherein the multi-objective function comprises a plurality of objective functions, wherein each objective function is configured to process the electronic representation of the molecule and to determine the strength of a specific pharmacological property of the molecule, and wherein said multi-objective function is particularly a combination of the plurality of objective functions,
- determining the drug score (101) of the modified molecule (190) by evaluating the multi-objective function for the modified molecule (190).

7. Method according to claim 6, wherein the objective functions are determined from a particularly supervised learning model, wherein said learning model is particularly a Quantitative Structure Activity Relationship (QSAR) or a Quantitative Structure Property Relationship (QSPR) model, and wherein objective functions for at least one of the following pharmacological properties, which may both be pharmacodynamic or pharmacokinetic properties, are modelled:

- therapeutic effect,
- side effects, particularly those related to the therapeutic effect
- toxicity, particularly cardiovascular toxicity or hepatoxicity, and/or
- Absorption, Distribution, Metabolism and Excretion.

8. Method according to one of the preceding claims, wherein the first moiety (110) of the selected molecule (100) comprises at least 1 heavy atom of the selected molecule (100), preferably all heavy atoms of the selected molecule (100), particularly more than 75% of the heavy atoms of the selected molecule (100), and most particularly more than 25% of the heavy atoms of the selected molecule (100).

9. Method according to one of the preceding claims, wherein the selected part (131) of the first moiety pharmacophore (130) comprises more than 10% of the molecular features of the first moiety pharmacophore (130), particularly more than 50% of the molecular features of the first moiety pharmacophore (130), more particularly more than 85% of the molecular features of the first moiety pharmacophore (130).

10. Method according to one of the preceding claims, wherein the selected part (131) of the first moiety pharmacophore (130) is increased when the drug score (101) of the modified molecule (190) is below the drug score (101) of the selected molecule (100) after particularly repeatedly executing steps g) to k).

11. Method according to one of the preceding claims, wherein additional graphical representations (132) of pharmacophore features are displayed on the graphical user interface (150).

12. Method according to claims 4, 6 or 7, wherein the plurality of modified molecules (190a, 190b, 190c) is further optimized performing the following steps:

- determining (300) a plurality of Pareto fronts (P1, P2) of the plurality of modified molecules (190a, 190b, 190c), wherein particularly the 10th best to best Pareto front (P1, P2) is determined and wherein the Pareto fronts (P1, P2) are determined with regard to the objective functions,
- ranking the molecules comprised by each Pareto (P1, P2) front either separately for each Pareto (P1, P2) front or jointly according to the strength of one of the specific pharmacological properties, particularly by the strength of the therapeutic effect,
- executing at least the steps a) to k) again, wherein the selected molecule (100) is one of the modified molecules (190a) that are comprised by the highest ranked modified molecules, particularly the highest ranked molecule.

13. Method according to claim 12, wherein the method further comprises the steps of:

- determining (304) for at least one of the highest ranked molecules (190a) a plurality of moieties (110a, 110b, 110c), wherein particularly the drug score from the respective molecule (190a) is assigned to each of the moieties of the plurality of moieties (110a, 110b, 110c) of the respective molecule (190a),
- determining (305) a molecular complexity (102) for each moiety of the plurality of moieties (110a, 110b, 110c) of the respective molecule (190a),
- particularly assigning (306) the molecule (190a) with the moiety associated to the highest complexity (102) as the selected molecule (100) and the respective moiety (110a) to the first moiety (110) of the selected molecule (100).

14. Computer program, comprising computer executable code that prompts a computer to execute the method according to one of the claims 1 to 13, when the computer program is run on a computer.

15. A system, particularly a computerized system, for designing a molecule for medical applications by optimization of an associated drug score of the molecule, the system comprising at least one client and a server operationally connected to the at least one client, wherein the server comprises:

a storage unit for storing a computer-readable representation of selected molecules, a drug score and the individual parts of the drug score associated to each of the selected molecule;
a processor operationally connected to the storage unit and configured to:

- select a molecule for display on a graphical user interface (150) of the at least one client,
- prompt to display the graphical representation of a selected part (131) of a first moiety pharmacophore (130) on the graphical user interface (150) of the client,
- prompt to display the graphical representations of the molecular building blocks (170) on the graphical user interface (150) of the at least one client,
- receive user input from the at least one client, wherein the user input is suited to instruct the client to rearrange the graphical representations of molecular building blocks on the display and save it on the server, wherein the input is provided by a user via an input unit such as a computer mouse, a computer keyboard, a touchscreen, a voice control unit, an accelerometer and/or any combinations thereof; and

wherein the client comprises:

- a processing unit configured to translate the user input into suitable commands for the server and to process data received from the server,
- a display unit configured to display the graphical user interface; and
- an input unit configured to obtain input data from the user, wherein the input data is configured to prompt the processor to arrange the graphical representations of the molecular building blocks.

**Patentansprüche**

1. Verfahren zur Gestaltung eines Moleküls für medizinische Anwendungen durch Optimierung eines assoziierten Wirkstoff-Scores (101) des Moleküls, wobei das Verfahren ein computerimplementiertes Verfahren ist, das durch eine computergestützte Vorrichtung durchgeführt wird, die einen Prozessor umfasst, umfassend die folgenden Schritte:

    a) Bereitstellen einer computerlesbaren Darstellung eines ausgewählten Moleküls (100) und eines mit dem ausgewählten Molekül (100) assoziierten Wirkstoff-Scores (101),
    b) Bestimmen (201) einer ersten Komponente (110) des ausgewählten Moleküls (100) und einer zweiten Komponente (120) des ausgewählten Moleküls (100), wobei das ausgewählte Molekül (100) aus der ersten Komponente (110) und der zweiten Komponente (120) besteht,
    c) Bestimmen (202) eines der ersten Komponente zugehörigen Pharmakophors (130), wobei die erste Komponente (110) des ausgewählten Moleküls (100) zu dem der ersten Komponente zugehörigen Pharmakophor (130) passt und wobei das der ersten Komponente zugehörige Pharmakophor anhand nur eines einzigen Moleküls bestimmt wird,
    d) Bereitstellen einer grafischen Benutzeroberfläche (150),
    e) Anzeigen (204) einer grafischen Darstellung eines ausgewählten Teils (131) des der ersten Komponente zugehörigen Pharmakophors (130) auf der grafischen Benutzeroberfläche (150),
    f) Bestimmen eines Startpunkts (160) auf der grafischen Benutzeroberfläche (150) in Bezug auf die grafische Darstellung des ausgewählten Teils (131) des der ersten Komponente zugehörigen Pharmakophors (130),
    g) ausgehend von dem Startpunkt (160), Anordnen von grafischen Darstellungen molekularer Bausteine (170) auf der grafischen Benutzeroberfläche (150), wobei die grafischen Darstellungen der molekularen Bausteine (170) miteinander verbunden sind und eine grafische Darstellung einer modifizierten ersten Komponente eines Moleküls (180) ausbilden,
    h) Zuordnen (207) der grafischen Darstellung der modifizierten ersten Komponente des Moleküls (180) zu einer modifizierten ersten Komponente (111) des ausgewählten Moleküls (100),
    i) Bestimmen (208) eines modifizierten Moleküls (190), das aus der modifizierten ersten Komponente (111) und der zweiten Komponente (120) des ausgewählten Moleküls (100) besteht,
    j) Schätzen (209) des assoziierten Wirkstoff-Scores (101) für das modifizierte Molekül (190),
    k) Offenlegen (210) des assoziierten Wirkstoff-Scores (101) des modifizierten Moleküls (190).

**2.** Verfahren nach Anspruch 1, wobei die Schritte g) bis k) wiederholt werden, bis der Wirkstoff-Score des modifizierten Moleküls (190) höher ist als der Wirkstoff-Score (101) des ausgewählten Moleküls (100).

**3.** Verfahren nach Anspruch 1 oder 2, wobei Schritt g) durch eine kollektive Intelligenz, insbesondere durch eine Mehrzahl von Benutzern, durchgeführt wird, wobei das Verfahren, insbesondere zeitgleich, auf einer Mehrzahl von Plattformen, insbesondere Computern, ausgeführt wird.

**4.** Verfahren nach Anspruch 3, wobei auf jeder Plattform ein modifiziertes Molekül (190) bestimmt wird, so dass eine Mehrzahl von modifizierten Molekülen (190a, 190b, 190c) mit erhöhtem Wirkstoff-Score (101) bestimmt wird.

**5.** Verfahren nach einem der vorangehenden Ansprüche, wobei eine Physiksimulationsmaschine bereitgestellt wird, wobei die Maschine die Position der grafischen Darstellungen der molekularen Bausteine (170), vorzugsweise ohne die Notwendigkeit einer menschliche Interaktion, gemäß ihren assoziierten molekularen Eigenschaften auf der grafischen Benutzeroberfläche (150) anpasst, wobei die assoziierten molekularen Eigenschaften insbesondere mindestens eine der folgenden umfassen:

    - Abstoßungs- und Anziehungskräfte zwischen den Darstellungen der molekularen Bausteine,
    - eine Ausschlussgröße der dargestellten molekularen Bausteine,
    - eine Länge der dargestellten molekularen Bausteine.

**6.** Verfahren nach einem der vorangehenden Ansprüche, wobei der Wirkstoff-Score (101) durch die folgenden Schritte bestimmt wird:

    - Bereitstellen einer Mehrzielfunktion, die dazu ausgestaltet ist, eine elektronische Darstellung eines Moleküls zu verarbeiten und den Wirkstoff-Score für das Molekül zu bestimmen, wobei die Mehrzielfunktion eine Mehrzahl von Zielfunktionen umfasst, wobei jede Zielfunktion dazu ausgestaltet ist, die elektronische Darstellung des Moleküls zu verarbeiten und die Stärke einer spezifischen pharmakologischen Eigenschaft des Moleküls zu bestimmen, und wobei die Mehrzielfunktion insbesondere eine Kombination der Mehrzahl von Zielfunktionen ist,
    - Bestimmen des Wirkstoff-Scores (101) des modifizierten Moleküls (190) durch Auswerten der Mehrzielfunktion für das modifizierte Molekül (190).

**7.** Verfahren nach Anspruch 6, wobei die Zielfunktionen anhand eines, insbesondere überwachten, Lernmodells bestimmt werden, wobei das Lernmodell insbesondere ein Quantitative-Structure-Activity-Relationship-Modell (QSAR-Modell) oder ein Quantitative-Structure-Property-Relationship-Modell (QSPR-Modell) ist und wobei Zielfunktionen für mindestens eine der folgenden pharmakologischen Eigenschaften, die sowohl pharmakodynamische als auch pharmakokinetische Eigenschaften sein können, modelliert werden:

- therapeutische Wirkung,
- Nebenwirkungen, insbesondere solche, die mit der therapeutischen Wirkung zusammenhängen,
- Toxizität, insbesondere kardiovaskuläre Toxizität oder Hepatoxizität, und/oder
- Aufnahme, Verteilung, Verstoffwechselung und Ausscheidung.

8. Verfahren nach einem der vorangehenden Ansprüche, wobei die erste Komponente (110) des ausgewählten Moleküls (100) mindestens ein Schweratom des ausgewählten Moleküls (100), vorzugsweise alle Schweratome des ausgewählten Moleküls (100), insbesondere mehr als 75 % der Schweratome des ausgewählten Moleküls (100) und ganz besonders mehr als 25 % der Schweratome des ausgewählten Moleküls (100) umfasst.

9. Verfahren nach einem der vorangehenden Ansprüche, wobei der ausgewählte Teil (131) des der ersten Komponente zugehörigen Pharmakophors (130) mehr als 10 % der molekularen Merkmale des der ersten Komponente zugehörigen Pharmakophors (130), insbesondere mehr als 50 % der molekularen Merkmale des der ersten Komponente zugehörigen Pharmakophors (130), ganz besonders mehr als 85 % der molekularen Merkmale des der ersten Komponente zughörigen Pharmakophors (130), umfasst.

10. Verfahren nach einem der vorangehenden Ansprüche, wobei der ausgewählte Teil (131) des der ersten Komponente zugehörigen Pharmakophors (130) erhöht wird, wenn der Wirkstoff-Score (101) des modifizierten Moleküls (190) nach insbesondere wiederholtem Ausführen der Schritte g) bis k) unter dem Wirkstoff-Score (101) des ausgewählten Moleküls (100) liegt.

11. Verfahren nach einem der vorangehenden Ansprüche, wobei auf der grafischen Benutzeroberfläche (150) zusätzliche grafische Darstellungen (132) von Pharmakophor-Merkmalen angezeigt werden.

12. Verfahren nach Anspruch 4, 6 oder 7, wobei die Mehrzahl von modifizierten Molekülen (190a, 190b, 190c) weiter optimiert wird, indem die folgenden Schritte durchgeführt werden:

- Bestimmen (300) einer Mehrzahl von Paretofronten (P1, P2) der Mehrzahl von modifizierten Molekülen (190a, 190b, 190c), wobei insbesondere die zehntbeste bis beste Paretofront (P1, P2) bestimmt werden und wobei die Paretofronten (P1, P2) in Bezug auf die Zielfunktionen bestimmt werden,
- Einstufen der zu jeder Paretofront (P1, P2) gehörenden Moleküle, entweder getrennt für jede Paretofront (P1, P2) oder zusammen gemäß der Stärke einer der spezifischen pharmakologischen Eigenschaften, insbesondere der Stärke der therapeutischen Wirkung,
- erneutes Ausführen zumindest der Schritte a) bis k), wobei das ausgewählte Molekül (100) eines der modifizierten Moleküle (190a), die zu den am höchsten eingestuften modifizierten Molekülen gehören, insbesondere das am höchsten eingestufte Molekül, ist.

13. Verfahren nach Anspruch 12, wobei das Verfahren ferner die folgenden Schritte umfasst:

- Bestimmen (304) einer Mehrzahl von Komponenten (110a, 110b, 110c) für mindestens eines der am höchsten eingestuften Moleküle (190a), wobei insbesondere der Wirkstoff-Score von dem jeweiligen Molekül (190a) jeder der Komponenten der Mehrzahl von Komponenten (110a, 110b, 110c) des jeweiligen Moleküls (190a) zugeordnet wird,
- Bestimmen (305) einer molekularen Komplexität (102) für jede Komponente der Mehrzahl von Komponenten (110a, 110b, 110c) des jeweiligen Moleküls (190a),
- insbesondere Zuordnen (306) des Moleküls (190a) mit der Komponente, die mit der höchsten Komplexität (102) assoziiert ist, als ausgewähltes Molekül (100) und der jeweiligen Komponente (110a) zu der ersten Komponente (110) des ausgewählten Moleküls (100).

14. Computerprogramm, umfassend computerausführbaren Code, der bewirkt, dass ein Computer das Verfahren nach einem der Ansprüche 1 bis 13 ausführt, wenn das Computerprogramm auf einem Computer ausgeführt wird.

15. System, insbesondere computergestütztes System, zum Gestalten eines Moleküls für medizinische Anwendungen durch Optimierung eines assoziierten Wirkstoff-Scores des Moleküls, wobei das System mindestens einen Client und einen mit dem mindestens einen Client wirkverbundenen Server umfasst, wobei der Server Folgendes umfasst:

eine Speichereinheit zum Speichern einer computerlesbaren Darstellung ausgewählter Moleküle, eines Wirkstoff-Scores und der einzelnen Teile des Wirkstoff-Scores, die mit jedem der ausgewählten Moleküle assoziiert sind;
einen Prozessor, der mit der Speichereinheit wirkverbunden und für Folgendes ausgestaltet ist:

- Auswählen eines Moleküls zur Anzeige auf einer grafischen Benutzeroberfläche (150) des mindestens einen Clients,
- Bewirken der Anzeige der grafischen Darstellung eines ausgewählten Teils (131) eines einer ersten Komponente zugehörigen Pharmakophors (130) auf der grafischen Benutzeroberfläche (150) des Clients,
- Bewirken der Anzeige der grafischen Darstellungen der molekularen Bausteine (170) auf der grafischen Benutzeroberfläche (150) des mindestens einen Clients,
- Empfangen einer Benutzereingabe von dem mindestens einen Client, wobei die Benutzereingabe dazu geeignet ist, den Client anzuweisen, die grafischen Darstellungen molekularer Bausteine auf der Anzeige neu anzuordnen und sie auf dem Server zu speichern, wobei die Eingabe durch einen Benutzer über eine Eingabeeinheit wie etwa eine Computermaus, eine Computertastatur, einen Touchscreen, eine Sprachsteuerungseinheit, einen Beschleunigungsmesser und/oder beliebige Kombinationen derselben bereitgestellt wird; und

wobei der Client Folgendes umfasst:

- eine Verarbeitungseinheit, die dazu ausgestaltet ist, die Benutzereingabe in geeignete Befehle für den Server zu übersetzen und vom Server empfangene Daten zu verarbeiten,
- eine Anzeigeeinheit, die dazu ausgestaltet ist, die grafische Benutzeroberfläche anzuzeigen; und
- eine Eingabeeinheit, die dazu ausgestaltet ist, Eingabedaten vom Benutzer zu erhalten, wobei die Eingabedaten dazu ausgestaltet sind zu bewirken, dass der Prozessor die grafischen Darstellungen der molekularen Bausteine anordnet.

## Revendications

1. Procédé de conception d'une molécule pour applications médicales par optimisation d'un score médicamenteux associé (101) de la molécule, dans lequel le procédé est un procédé mis en oeuvre par ordinateur réalisé par un dispositif informatisé comprenant un processeur, comprenant les étapes consistant à :

a) fournir une représentation lisible par ordinateur d'une molécule sélectionnée (100) et d'un score médicamenteux (101) associé à la molécule sélectionnée (100),
b) déterminer (201) un premier groupe caractéristique (110) de la molécule sélectionnée (100) et un second groupe caractéristique (120) de la molécule sélectionnée (100), dans lequel la molécule sélectionnée (100) est constituée du premier groupe caractéristique (110) et du second groupe caractéristique (120),
c) déterminer (202) un pharmacophore de premier groupe caractéristique (130), dans lequel le premier groupe caractéristique (110) de la molécule sélectionnée (100) s'adapte au pharmacophore de premier groupe caractéristique (130), et dans lequel le pharmacophore de premier groupe caractéristique est déterminé à partir d'une seule molécule uniquement,
d) fournir une interface utilisateur graphique (150),
e) afficher (204) une représentation graphique d'une partie sélectionnée (131) du pharmacophore de premier groupe caractéristique (130) sur l'interface utilisateur graphique (150),
f) déterminer un point de départ (160) sur l'interface utilisateur graphique (150) par rapport à la représentation graphique de la partie sélectionnée (131) du pharmacophore de premier groupe caractéristique (130),
g) à partir du point de départ (160), agencer des représentations graphiques de blocs de construction moléculaire (170) sur l'interface utilisateur graphique (150), dans lequel les représentations graphiques des blocs de construction moléculaire (170) sont interconnectées et forment une représentation graphique d'un premier groupe caractéristique modifié d'une molécule (180),
h) attribuer (207) la représentation graphique du premier groupe caractéristique modifié de la molécule (180) à un premier groupe caractéristique modifié (111) de la molécule sélectionnée (100),
i) déterminer (208) une molécule modifiée (190) constituée du premier groupe caractéristique modifié (111) et du second groupe caractéristique (120) de la molécule sélectionnée (100),
j) estimer (209) le score médicamenteux associé (101) pour la molécule modifiée (190),
k) divulguer (210) le score médicamenteux associé (101) de la molécule modifiée (190).

**2.** Procédé selon la revendication 1, dans lequel les étapes g) à k) sont répétées jusqu'à ce que le score médicamenteux de la molécule modifiée (190) soit supérieur au score médicamenteux (101) de la molécule sélectionnée (100).

**3.** Procédé selon la revendication 1 ou 2, dans lequel l'étape g) est réalisée par une intelligence collective, notamment par une pluralité d'utilisateurs, dans lequel le procédé est exécuté, notamment simultanément, sur une pluralité de plates-formes, notamment d'ordinateurs.

**4.** Procédé selon la revendication 3, dans lequel sur chaque plate-forme, une molécule modifiée (190) est déterminée de sorte qu'une pluralité de molécules modifiées (190a, 190b, 190c) avec un score médicamenteux accru (101) est déterminée.

**5.** Procédé selon une des revendications précédentes, dans lequel un moteur de simulation de physique est fourni, dans lequel ledit moteur ajuste la position des représentations graphiques des blocs de construction moléculaire (170), de préférence sans la nécessité d'une quelconque interaction humaine, en fonction de leurs propriétés moléculaires associées sur l'interface utilisateur graphique (150), dans lequel les propriétés moléculaires associées comprennent notamment au moins une de :

- forces de répulsion et d'attraction entre les représentations des blocs de construction moléculaire,
- une taille d'exclusion des blocs de construction moléculaire représentés,
- une longueur des blocs de construction moléculaire représentés.

**6.** Procédé selon une des revendications précédentes, dans lequel le score médicamenteux (101) est déterminé par les étapes consistant à :

- fournir une fonction multi-objectif qui est configurée pour traiter une représentation électronique d'une molécule et pour déterminer le score médicamenteux pour ladite molécule, dans lequel la fonction multi-objectif comprend une pluralité de fonctions objectifs, dans lequel chaque fonction objectif est configurée pour traiter la représentation électronique de la molécule et pour déterminer la force d'une propriété pharmacologique spécifique de la molécule, et dans lequel ladite fonction multi-objectif est notamment une combinaison de la pluralité de fonctions objectifs,
- déterminer le score médicamenteux (101) de la molécule modifiée (190) en évaluant la fonction multi-objectif pour la molécule modifiée (190).

**7.** Procédé selon la revendication 6, dans lequel les fonctions objectifs sont déterminées à partir d'un modèle d'apprentissage particulièrement supervisé, dans lequel ledit modèle d'apprentissage est notamment un modèle de relation quantitative structure-activité (QSAR) ou un modèle de relation quantitative structure-propriété (QSPR), et dans lequel des fonctions objectifs pour au moins une des propriétés pharmacologiques suivantes, qui peuvent être à la fois des propriétés pharmacodynamiques ou pharmacocinétiques, sont modélisées :

- effet thérapeutique,
- effets secondaires, notamment ceux liés à l'effet thérapeutique,
- toxicité, notamment toxicité cardiovasculaire ou hépatotoxicité, et/ou
- absorption, distribution, métabolisme et excrétion.

**8.** Procédé selon une des revendications précédentes, dans lequel le premier groupe caractéristique (110) de la molécule sélectionnée (100) comprend au moins 1 atome lourd de la molécule sélectionnée (100), de préférence tous les atomes lourds de la molécule sélectionnée (100), en particulier plus de 75 % des atomes lourds de la molécule sélectionnée (100), et plus particulièrement plus de 25 % des atomes lourds de la molécule sélectionnée (100).

**9.** Procédé selon une des revendications précédentes, dans lequel la partie sélectionnée (131) du pharmacophore de premier groupe caractéristique (130) comprend plus de 10 % des caractéristiques moléculaires du pharmacophore de premier groupe caractéristique (130), en particulier plus de 50 % des caractéristiques moléculaires du pharmacophore de premier groupe caractéristique (130), plus particulièrement plus de 85 % des caractéristiques moléculaires du pharmacophore de premier groupe caractéristique (130).

**10.** Procédé selon une des revendications précédentes, dans lequel la partie sélectionnée (131) du pharmacophore de premier groupe caractéristique (130) est augmentée lorsque le score médicamenteux (101) de la molécule modifiée (190) est en dessous du score médicamenteux (101) de la molécule sélectionnée (100) après l'exécution particulièrement répétée des étapes g) à k).

**11.** Procédé selon une des revendications précédentes, dans lequel des représentations graphiques supplémentaires (132) des caractéristiques de pharmacophore sont affichées sur l'interface utilisateur graphique (150).

**12.** Procédé selon les revendications 4, 6 ou 7, dans lequel la pluralité de molécules modifiées (190a, 190b, 190c) est en outre optimisée en réalisant les étapes suivantes :

- déterminer (300) une pluralité de fronts de Pareto (P1, P2) de la pluralité de molécules modifiées (190a, 190b, 190c), dans lequel le 10e front de Pareto de meilleur à meilleur (P1, P2) est déterminé et dans lequel les fronts de Pareto (P1, P2) sont déterminés par rapport aux fonctions objectifs,
- classer les molécules comprises par chaque front de Pareto (P1, P2) soit séparément pour chaque front de Pareto (P1, P2), soit de manière conjointe en fonction de la force d'une des propriétés pharmacologiques spécifiques, notamment par la force de l'effet thérapeutique,
- exécuter au moins les étapes a) à k) de nouveau, dans lequel la molécule sélectionnée (100) est une des molécules modifiées (190a) qui sont comprises par les molécules modifiées au classement le plus haut, notamment la molécule au classement le plus haut.

13. Procédé selon la revendication 12, dans lequel le procédé comprend en outre les étapes consistant à :

- déterminer (304) pour au moins une des molécules à classement le plus haut (190a) une pluralité de groupes caractéristiques (110a, 110b, 110c), dans lequel notamment le score médicamenteux provenant de la molécule respective (190a) est attribué à chacun des groupes caractéristiques de la pluralité de groupes caractéristiques (110a, 110b, 110c) de la molécule respective (190a),
- déterminer (305) une complexité moléculaire (102) pour chaque groupe caractéristique de la pluralité de groupes caractéristiques (110a, 110b, 110c) de la molécule respective (190a),
- notamment assigner (306) la molécule (190a) avec le groupe caractéristique associé à la complexité la plus élevée (102) en tant que la molécule sélectionnée (100) et le groupe caractéristique respectif (110) au premier groupe caractéristique (110) de la molécule sélectionnée (100).

14. Programme informatique, comprenant du code exécutable par ordinateur qui invite un ordinateur à exécuter le procédé selon une des revendications 1 à 13 lorsque le programme informatique est exécuté sur un ordinateur.

15. Système, notamment système informatisé, de conception d'une molécule pour applications médicales par optimisation d'un score médicamenteux associé de la molécule, le système comprenant au moins un client et un serveur connecté de manière opérationnelle à l'au moins un client, dans lequel le serveur comprend :

un module de stockage pour stocker une représentation lisible par ordinateur de molécules sélectionnées, un score médicamenteux et les parties individuelles du score médicamenteux associé à chacune de la molécule sélectionnée ;
un processeur connecté de manière opérationnelle au module de stockage et configuré pour :

- sélectionner une molécule pour l'affichage sur une interface utilisateur graphique (150) de l'au moins un client,
- inviter à afficher la représentation graphique d'une partie sélectionnée (131) d'un pharmacophore de premier groupe caractéristique (130) sur l'interface utilisateur graphique (150) du client,
- inviter à afficher les représentations graphiques des blocs de construction moléculaire (170) sur l'interface utilisateur graphique (150) de l'au moins un client,
- recevoir une saisie d'utilisateur de l'au moins un client, dans lequel la saisie d'utilisateur convient à instruire le client de réagencer les représentations graphiques de blocs de construction moléculaire sur l'écran et les sauvegarder sur le serveur, dans lequel la saisie est fournie par un utilisateur par le biais d'un module de saisie tel qu'une souris, un clavier, un écran tactile, un module de commande vocale, un accéléromètre et/ou toute combinaison de ceux-ci ; et

dans lequel le client comprend :

- un module de traitement configuré pour traduire la saisie d'utilisateur en commandes appropriées pour le serveur et pour traiter des données reçues du serveur,
- un module d'affichage configuré pour afficher l'interface utilisateur graphique ; et
- un module de saisie configuré pour obtenir des données saisies de l'utilisateur, dans lequel les données saisies sont configurées pour inviter le processeur à agencer les représentations graphiques des blocs de construction moléculaire.

Fig. 1

Fig. 2

Fig.3

150

500

503

501

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Computer-based de novo design of drug-like molecules. **SCHNEIDER G. et al.** NATURE REVIEWS. DRUG DISCOVERY. NATURE PUBLISHING GROUP, 01 August 2005, vol. 4, 649-663
- An approach to intelligent drug design support. **SMITHERS T. et al.** SYSTEM SCIENCES, 1993, PROCEEDING OF THE TWENTY-SIXTH HAWAII INTERNATIONAL CONFERENCE ON WAILEA. IEEE, 05 January 1993, 634-645
- **BOHACEK** ; **MCMARTIN** ; **GUIDA**. The Art and Practice of Structure-Based Drug Design A Molecular Modeling Perspective. *Medicinal research reviews*, 1996
- **TROPSHA A**. Best Practices for QSAR Model Development, Validation, and Explotation. *Mol. Inf.*, 2010, vol. 29, 476-488
- **ERIKSSON L. et al.** Methods for Reliability and Uncertainty Assessment and for Applicability Evaluations of Classification- and Regression-Based QSARs. *EHP*, 2003, vol. 111 (10), 1361-1375